# EUROPÄISCHE PATENTSCHRIFT

(11) **EP 0 811 607 B1**
(45) Veröffentlichungstag und Bekanntmachung des Hinweises auf die Patenterteilung: **08.09.1999**
(21) Anmeldenummer: 97105739.3
(22) Anmeldetag: 08.04.1997
(51) Int. Cl.: C07C 249/06

(54) **Verfahren zur Nitrosierung von C-H-aciden Verbindungen**
Process for the nitrosation of C-H- acid compounds
Procédé de nitrosation de composés à C-H- acides

(30) Priorität: 05.06.1996 DE 19622467
(43) Veröffentlichungstag der Anmeldung: 10.12.1997
(73) Patentinhaber: HÜLS AKTIENGESELLSCHAFT, 45764 Marl (DE)
(72) Erfinder: Bauer, Frank, Dr., 51143 Köln (DE); Feld, Marcel, Dr., 51147 Köln (DE)

(56) Entgegenhaltungen:
- EP-A- 0 174 519
- EP-A- 0 483 674
- DE-A- 2 352 706
- DE-C- 954 873
- PATENT ABSTRACTS OF JAPAN vol. 10, no. 239 (C-367) [2295] , 19.August 1986 & JP 61 072741 A (NIPPON KAYAKU CO LTD), 14.April 1986,

## Beschreibung

Die Anmeldung betrifft die Nitrosierung von C-H-aciden Verbindungen der allgemeinen Formel X¹-CHR.-X² (I), in der R Wasserstoff oder einen organischen Rest bedeutet und X¹ sowie X² gleich oder verschieden sein können und für elektronenanziehende Gruppen stehen.

Nitrosierungsprodukte C-H-Acider Verbindungen werden u.a. als Vorprodukte für die Herstellung der entsprechenden Amine verwendet, die ihrerseits für die Synthese verschiedener Pharmazeutika und Wirkstoffe für Pflanzenschutzmittel benötigt werden. Als Beispiele seien Hydroximino-und Nitrosoverbindungen von Malonsäurederivaten genannt. Es ist eine Reihe von Verfahren zur Nitrosierung von Malonsäurederivaten, wie Estern, Amiden, Imidoestern oder Malodinitril, bekannt geworden. J.B.Paine III et al. beschreiben in J.Org.Chem.50 (1985),5598-5604 ein Verfahren zur Herstellung des Hydroximinomalonsäurediethylesters, bei dem man einer Lösung von Malonsäurediethylester in Eisessig langsam eine wäßrige Lösung von Natriumnitrit zusetzt, dem Reaktionsgemisch, das eine homogene Lösung ist, Natronlauge zufügt und das Reaktionsprodukt durch Extraktion mit Diethylether von der wäßrigen, Natriumacetat enthaltenden Phase abtrennt. Bei dem Verfahren fällt Natriumacetat als wäßrige Lösung und in der rund 4-fachen molaren Menge, bezogen auf Malonsäurediethylester, an.

Nach der DE-OS-2 352 706 wird Cyanessigsäureethylester zunächst mit Chlorwasserstoff in absolutem Alkohol in Monoimidomalonsäurediethylester-hydrochlorid umgewandelt und dieses in Essigsäure gelöst. Der Lösung wird dann nach und nach eine wäßrige Lösung von Natriumnitrit zugefügt, und dem Reaktionsgemisch wird nach Beendigung der Nitrosierungsreaktion Wasser zugesetzt. Das Reaktionsprodukt wird von der wäßrigen Phase, die das entstandene Natriumacetat enthält, wiederum durch Extraktion mit einem Lösungsmittel abgetrennt.

In der EP-A1-0 517 041 findet sich ein Beispiel für die Herstellung von Hydroximinomalonsäuredimethylester, bei dem einem Gemisch aus Malonsäuredimethylester und Wasser Natriumnitrit und Essigsäure zugesetzt wird. Das Reaktionsgemisch wird zweimal mit Dichlorethan extrahiert und der Hydroximinomalonsäurediethylester so von dem Natriumacetat getrennt, das in der wäßrigen Phase verbleibt. Dabei wird zwar das Natriumnitrit nur in Mengen von 1,2 Mol pro Mol Malonsäureester angewandt, jedoch machen die Reaktionszeiten von 21 Stunden die Umsetzung für ein technisches Verfahren praktisch unbrauchbar. Außerdem eignet sich das Verfahren nicht für die Umsetzung von wenig wasserlöslichen Malonsäureestern.

Bei allen aufgeführten Verfahren fällt das Natriumacetat in Form verunreinigter, schwer zu entsorgender wäßriger Lösungen an. Die Verfahren sind daher aus ökologischen Gründen für eine Übertragung in den technischen Maßstab ungeeignet.

In DE 954 873 wird ein Verfahren zur Herstellung von Hydroximinomalonsäurediethylester beschrieben, bei dem man Malonsäurediethylester in einem mit Wasser nicht merklich mischbaren, vom Endprodukt durch Destillation abtrennbaren Lösungsmittel, wie Toluol, löst, dieser Lösung mindestens molare Mengen Natriumnitrit sowie 1 bis 10 Gewichtsprozent, bezogen auf den Malonsäureester, Wasser zufügt, der Suspension bei einer Temperatur von 30 bis 70°C allmählich Eisessig zusetzt bis die Nitrosierung beendet ist, die Reaktionslösung von ungelöstem Natriumacetat abtrennt und aus der Lösung kristallisierten Hydroximinomalonsäurediethylester gewinnt. Dieses Verfahren kommt ohne Lösungsmittelextraktion aus, und es werden immerhin etwa 2/3 des Natriumacetats in fester Form gewonnen. Das Verfahren soll "glatte und schnelle Umsetzungen und gute Ausbeuten" ergeben. Zumindest letzteres trifft jedoch nicht zu, weil das erhaltene kristalline Produkt vom Schmelzpunkt 86,5-88°C gar nicht der Hydroximinomalonsäurediethylester, sondern dessen Komplex mit Natriumacetat war. Das Produkt ist so unrein, daß die Hydrierung zum Acetaminomalonsäurediethylester an Platinkatalysatoren in dem als Lösungsmittel besonders vorteilhaften Essigsäureanhydrid nicht möglich ist.

Es bestand daher die Aufgabe, ein Verfahren bereitzustellen, nach dem sich C-H-acide Verbindungen, auch und insbesondere solche, die wenig wasserlöslich sind, mit einem möglichst geringen Überschuß an Alkalinitrit in möglichst kurzer Reaktionszeit bei hohem Umsatz und mit guten Ausbeuten zu Hydroximino- oder Nitrosoverbindungen mit der für Folgereaktionen erforderlichen hohen Reinheit umsetzen lassen, wobei die als Nebenprodukte anfallenden Salze möglichst weitgehend in fester, wiederverwendbarer Form gewonnen werden und der Anfall von stark verunreinigtem Abwasser weitgehend oder vollständig vermieden wird.

Es wurde nun gefunden, daß sich C-H-acide Verbindungen der allgemeinen Formel

X¹-CHR-X², (I)

in der R Wasserstoff oder einen organischen Rest bedeutet und X¹ sowie X² für gleiche oder verschiedene elektronenanziehende Gruppen stehen, durch Umsetzung mit salpetriger Säure, die aus einem Nitrit durch eine andere Säure freigesetzt wird, in Gegenwart von Wasser und eines inerten organischen Lösungsmittels vorteilhaft nitrosieren lassen, wenn man als inertes organisches Lösungsmittel ein mit Wasser zumindest teilweise mischbares inertes organisches Lösungsmittel oder Lösungsmittelgemisch verwendet und die Mengen dieses Lösungsmittels oder Lösungsmittelgemisches und des Wassers so aufeinander abstimmt, daß der Ausgangsstoff (I), dessen Nitrosierungsprodukt, das Wasser sowie das inerte organische Lösungsmittel und zumindest ein Teil des Nitrits und der anderen Säure, die die salpetrige Säure freisetzt, eine homogene flüssige Phase bilden, in der die Nitrosierungsreaktion abläuft und aus der zumindest der größte Teil des bei der Freisetzung der salpetrigen Säure entstehenden Salzes ausfällt.

In bevorzugten C-H-aciden Verbindungen der allgemeinen Formel (I) bedeutet R Wasserstoff. Die Nitrosierungsprodukte fallen in diesem Fall nicht als Nitrosoverbindungen, sondern in Form der tautomeren Hydroximinoverbindungen an. Wenn die andere Säure, die aus dem Nitrit salpetrige Säure freisetzt, eine Carbonsäure ist, entstehen daneben in wechselnden Mengen auch die entsprechenden O-acylierten Hydroxyminoverbindungen. Die Nitrosierungsprodukte enthalten dann also die Gruppe C=NOR', in der R' Wasserstoff oder einen Acylrest bedeutet. In anderen geeigneten Ausgangsstoffen (I) bedeutet R einen aliphatischen Rest, z.B. einen Alkylrest mit 1 bis 4 Kohlenstoffatomen, oder einen aromatischen Rest, z.B. einen Arylrest mit 6 bis 10 Kohlenstoffatomen.

Die elektronenanziehenden Gruppen X¹ und X² können gleich oder verschieden sein. X¹ und X² stehen vorzugsweise für die Gruppen -COOR, -C(NR)OR, -CONR₂, -CO-R, wobei R die für die Formel (I) angegebene Bedeutung, einschließlich der bevorzugten Bedeutungen hat, sowie für die Gruppen -CN, -NO₂ oder aromatische Reste, insbesondere für Arylreste mit 6 bis 10 Kohlenstoffatomen, die durch inerte Gruppen oder Atome substituiert sein können. Von den als Ausgangsstoffe für das Verfahren nach der Erfindung geeigneten C-H-aciden Verbindungen seien z.B. genannt: Malonsäure sowie deren Ester und Imidoester, wie Malonsäuredimethylester, 2-Methylmalonsäuredimethylester, Malonsäurediethylester, 2-Phenylmalonsäurediethylester, Malonsäurediisobutylester, Malonsäuredi-2-ethylhexylester und Monoimidomalonsäurediethylester; Malonsäureamide oder -amidoester, wie Malonsäurediamid, N,N' -Dimethylmalonsäurediamid, N'N'N',N'-Tetramethylmalonsäurediamid und N,N-Dimethylamidomalonsäureethylester; Malonsäuredinitril; Cyanessigsäure und deren Ester, wie Cyanessigsäureethylester; 2-Cyanpropionsäure und deren Ester, wie 2-Cyanpropionsäureethylester; beta-Ketosäuren und deren Derivate, wie Acetessigsäure oder Benzoylessigsäure und deren Ester oder Amide, z.B. Acetessigsäureethylester, Acetessigsäure-N,N-dimethylamid und Benzoylessigsäureethylester; 1,3-Diketone, wie Acetylaceton, Benzoylaceton und Dibenzoylmethan ; Nitroverbindungen, wie Dinitromethan, Nitroessigsäureethylester und Nitroacetonitril; aromatische Verbindungen mit einer weiteren die Gruppe -CHR- flankierenden elektronenanziehenden Gruppe, wie Phenylessigsäureester, Phenylacetontril (Benzylnitril) und p-Nitrophenylacetonitril.

Das eigentliche Nitrosierungsmittel, die salpetrige Säure, wird in situ aus einem Nitrit durch eine andere Säure freigesetzt, Geeignete Nitrite sind insbesondere Alkalinitrite, wie Natrium- und Kaliumnitrit, die im allgemeinen in Mengen von bis zu 3 Mol je Mol C-H-acide Verbindung (1) verwendet werden. Zweckmäßig setzt man das Nitrit in Mengen von 1 bis 1,5 Mol, insbesondere von 1,05 bis 1,2 Mol je Mol C-H-acide Verbindung ein.

Als die andere Säure, die die salpetrige Säure aus dem Nitrit freisetzt, eignet sich jede anorganische oder organische Säure, die das Nitrit wenigstens partiell zu protonieren vermag, Geeignete anorganische Säuren sind u.a. Salzsäure, Schwefelsäure, Salpetersäure und Phosphorsäure. Von den geeigneten organischen Säuren seien insbesondere Carbonsäuren, beispielsweise Ameisensäure, Essigsäure und Propionsäure, genannt. Besonders bevorzugt werden Schwefelsäure und Essigsäure. Man wendet die Säure vorteilhaft in mindestens der dem Nitrit entsprechenden stöchiometrischen Menge an. Größere Mengen an Säuren sind nicht schädlich, und größere Mengen an Carbonsäuren sind insbesondere dann empfehlenswert, wenn das Nitrosierungsprodukt anschließend ohne vorherige Abtrennung oder Reinigung zum Amin hydriert werden soll. Man kann die Säure auch ganz oder teilweise durch ein Säureanhydrid ersetzen. Dies empfiehlt sich besonders, wenn die C-H-acide Verbindung und/oder deren Nitrosierungsprodukt nicht gut in Wasser bzw. wasserreichen Reaktionsgemischen löslich sind. Das Säureanhydrid fängt dann das bei der Nitrosierungsreaktion entstehende Wasser ab, so daß sich das Reaktionsgemisch nicht in unerwünschtem Maße mit Wasser anreichert. Natürlich darf das Säureanhydrid nicht in so großen Mengen zugegen sein, daß es auch das zugesetzte Wasser verbraucht, weil dann die Löslichkeit des Nitrits im Reaktionsmedium, also der erwähnten homogenen flüssigen Phase, für eine praktisch brauchbare Reaktionsgeschwindigkeit zu gering wäre.

Wasser ist nämlich ein unverzichtbarer Bestandteil des Reaktionsgemisches, da es die Freisetzung von salpetriger Säure aus dem Nitrit ermöglicht und fördert. Eine bestimmte, von den Gegebenheiten des Einzelfalls abhängige Mindestmenge Wasser sollte vorhanden sein, damit ein möglichst vollständiger und schneller Umsatz bei mäßig hohen Temperaturen erreicht wird. Wird weniger Wasser verwendet, so kann ein Teil des Nitrits unumgesetzt bleiben und die Entsorgung bzw. Wiederverwendung des bei der Reaktion gebildeten Salzes erschweren. Andererseits sollte das Wasser in möglichst geringer Menge insbesondere dann vorhanden sein, wenn die C-H-acide Verbindung und/oder deren Nitrosierungsprodukt schlecht wasserlöslich sind. Beiden Forderungen kann man im allgemeinen genügen, wenn man 0,01 bis 50, vorzugsweise 0,03 bis 20 Gewichtsprozent Wasser, bezogen auf das inerte organische, mit Wasser zumindest teilweise mischbare organische Lösungsmittel oder Lösungsmittelgemisch, mitverwendet.

Die Mitverwendung eines solchen Lösungsmittels oder Lösungsmittelgemisches ist ein wichtiges Merkmal des Verfahrens nach der Erfindung. Sie ermöglicht eine vorteilhafte Nitrosierung auch und insbesondere von wenig wasserlöslichen C-H-aciden Verbindungen, wie Malonsäureestern, beta-Ketosäureestern und höheren beta-Diketonen. Das Lösungsmittel oder Lösungsmittelgemisch trägt dazu bei, daß sich eine homogene Phase ausbildet, in der alle Bestandteile des Reaktionsgemisches vertreten sind, in der die Nitrosierungsreaktion abläuft und aus der das bei der Nitrosierungsreaktion gebildete Salz weitgehend ausfällt. In dieser Phase muß nicht das gesamte Nitrit gelöst vorliegen. Es genügt, wenn ein Teil davon gelöst wird, so daß eine für eine schnelle Umsetzung genügende Menge salpetrige Säure freigesetzt werden kann, während eine andere Teilmenge suspendiert bleibt und nach und nach in dem Maße in Lösung geht, in dem das gelöste Nitrit in der Nitrosierungsreaktion verbraucht wird. Diese Bedingung ist erfüllt, wenn die zuvor angegebenen Mengen an an Wasser mitverwendet werden. Die Menge der im Reaktionsmedium, also in der erwähnten homogenen flüssigen Phase, gelösten anderen Säure, die aus dem Nitrit salpetrige Säure freisetzt, muß natürlich mindestens der Menge des gelösten Nitrits entsprechen. Da jedoch die andere Säure im Reaktionsgemisch im allgemeinen deutlich besser löslich ist als das Nitrit, gibt es insoweit keine Probleme, und die andere Säure ist in der Regel vollständig gelöst. Dies gilt natürlich besonders für die später erläuterten Ausführungsformen, in denen die Säure zudosiert wird, im allgemeinen aber auch für die Varianten, in denen die Säure mit vorgelegt wird.

Unter zumindest teilweise mit Wasser mischbaren organischen Lösungsmitteln werden im Zusammenhang mit der Erfindung insbesondere solche verstanden, die bei Raumtemperatur mindestens 1 Gewichtsprozent, vorteilhaft mindestens 3 Gewichtsprozent und insbesondere mehr als 5 Gewichtsprozent Wasser zu lösen vermögen. Vielfach sind die organischen Lösungsmittel mit Wasser in jedem Mengenverhältnis zu homogenen Lösungen mischbar. Lösungsmittelgemische sind in gleicher Weise geeignet, sofern sie die angegebene Mindestlöslichkeit für Wasser aufweisen. Man kann also mit einem Gemisch aus einem Lösungsmittel arbeiten, das keine merklichen Mengen Wasser löst, und einem anderen. Wasser gut lösenden oder mit Wasser gut mischbaren Lösungsmittel, sofern dieses Gemisch Wasser in den angegebenen Mindestmengen löst. Das inerte organische Lösungsmittel oder Lösungsmittelgemisch wird zweckmäßig in Mengen von 0,05 bis 2,5 Gewichtsteilen, vorteilhaft von 0,35 bis 2,0 Gewichtsteilen je Gewichtsteil C-H-acider Verbindung eingesetzt. Natürlich können auch größere Mengen an inertem organischen Lösungsmittel oder Lösungsmittelgemisch verwendet werden, wodurch jedoch die Raum-Zeit-Ausbeute vermindert wird.

Geeignete inerte organische Lösungsmittel sind z.B. aliphatische oder cyclische Ether, wie Dibutylether, Methyl-tert.butylether, Tetrahydrofuran, 1,4-Dioxan, Dialkoxyalkane (z.B. 1,2-Dimethoxyethan) und Polyethylenglykolether; Carbonsäureester, insbesondere aus Carbonsäuren und Alkoholen mit jeweils 1 bis 4 Kohlenstoffatomen, wie Methylacetat und Ethylacetat; und N,N-disubstituierte Carbonsäureamide, insbesondere von Carbonsäuren mit 1 bis 4 Kohlenstoffatomen, wie N,N-Dimethylformamid und N,N-Dimethylacetamid; sowie Nitrile, wie Acetonitril und Propionitril. Ein bevorzugtes Lösungsmittel ist 1,4-Dioxan. Wenn zur Freisetzung der salpetrigen Säure eine organische Säure, wie Ameisensäure. Essigsäure und Propionsäure, verwendet wird, so kann diese Säure zugleich als Lösungsmittel dienen. Dies ist möglich, sofern die Mengen der organischen Säure und der anderen Komponenten des Reaktionsgemisches so gewählt werden, daß einerseits die Nitrosierungsreaktion in homogener Phase abläuft und andererseits zumindest der größte Teil des bei der Freisetzung der salpetrigen Säure entstehenden Salzes ausfällt.

Nach einem wesentlichen Merkmal des Verfahrens nach der Erfindung werden nämlich die Mengen des inerten, zumindest teilweise mit Wasser mischbaren organischen Lösungsmittels oder Lösungsmittelgemisches und des Wassers so aufeinander abgestimmt, daß als Reaktionsmedium eine homogene flüssige Phase entsteht, die einerseits die C-H-acide Verbindung und deren Nitrosierungsprodukt vollständig sowie das Nitrit und die Säure, die daraus salpetrige Säure freisetzt, zumindest teilweise löst, und andererseits zumindest der größte Teil, vorteilhaft mindestens 60 Gewichtsprozent und insbesondere mindestens 80 Gewichtsprozent, des bei der Nitrosierungsreaktion entstehenden Salzes in fester Form ausfällt. Dabei kann der Fachmann den Zielkonflikt für eine bestimmte Nitrosierungsaufgabe - genügend Wasser zu verwenden, damit sich das Nitrit und die Säure, die daraus salpetrige Säure freisetzt, hinreichend lösen, aber möglichst wenig Wasser, damit das bei der Nitrosierungsreaktion entstehende Salz möglichst weitgehend ausfällt - bei Beobachtung der zuvor angegebenen Mengen mittels orientierender Versuche unschwer optimal lösen. Jedenfalls sollte der größte Teil des Salzes ausfallen und nicht als wässerige Lösung anfallen.

Das Verfahren nach der Erfindung wird vorteilhaft im Temperaturbereich von 0 bis 100°C durchgeführt. Zweckmäßig arbeitet man im Bereich von 20 bis 60°C, insbesondere von 30 bis 40°C, Unterhalb von 0°C verläuft die Reaktion sehr langsam, oberhalb von 100°C kann Zersetzung eintreten und/oder können Nebenprodukte entstehen. Es empfiehlt sich, die Temperatur gegen Ende der Reaktion innerhalb der angegebenen Grenzen zu steigern, um den Umsatz zu vervollständigen. Man arbeitet in der Regel bei Atmosphärendruck. Bei höheren Reaktionstemperaturen und Verwendung leicht siedender inerter organischer, zumindest teilweise mit Wasser mischbaren Lösungsmitteln, wie Methylacetat, kann es ratsam sein, erhöhten Druck anzuwenden, damit das Lösungsmittel nicht entweicht.

Zur Durchführung des Verfahrens nach der Erfindung kann man das inerte organische Lösungsmittel, das Wasser, das Nitrit und die C-H-acide Verbindung in einem Rührreaktor vorlegen und nach und nach die Säure zufügen, die die salpetrige Säure freisetzt, Alternativ kann man die C-H-acide Verbindung, das inerte organische Lösungsmittel, Wasser und die Säure vorlegen und portionsweise das Nitrit zusetzen. Diese Arbeitsweise ist empfehlenswert, wenn die Carbonsäure, die aus dem Nitrit die salpetrige Säure freisetzt, zugleich als inertes organisches Lösungsmittel dient. Bei beiden Varianten ist es möglich, das Wasser nicht mit vorzulegen, sondern nach und nach separat oder zusammen mit der Säure bzw. dem Nitrit zuzuführen. Schließlich ist es auch möglich, alle genannten Komponenten außer dem Wasser vorzulegen und dieses nach und nach zuzusetzen. Wie bereits erwähnt, kann die Säure zumindest teilweise durch ein Anhydrid ersetzt werden, damit der Wassergehalt der Reaktionsphase nicht unerwünscht ansteigt. Dies gilt besonders dann, wenn die Säure nicht vorgelegt, sondern nach und nach zugesetzt wird.

Bei allen Varianten wird gegebenenfalls gekühlt, um die gewünschte Reaktionstemperatur einzuhalten. Die Reaktionszeit hängt von der Art der C-H-aciden Verbindung, der Reaktionstemperatur, der Kühlkapazität sowie anderen Gegebenheiten ab. Zweckmäßig steigert man die Temperatur gegen Ende der Säurezugabe um 10 bis 20°C und läßt bei dieser Temperatur nachreagieren. Dadurch werden die letzten Reste an Nitrit zersetzt, so daß das entstehende ausgefallene Salz praktisch frei davon ist, und verbliebene nitrose Gase werden aus dem Reaktionsgemisch ausgetrieben. Letzteres kann man u.a. dadurch erreichen, daß man ein Inertgas, wie Stickstoff durchleitet. Bei den bevorzugten Temperaturen von 20 bis 60°C nimmt die Reaktion im allgemeinen bis zu etwa 2 Stunden in Anspruch.

Das bei der Nitrosierungsreaktion entstehende Salz bleibt um so weniger in der Reaktionsphase gelöst, je geringer deren Wassergehalt ist. Man kann diesen Wassergehalt nachträglich vermindern, z.B. indem man dem Reaktionsgemisch ein wasserfreies Salz zusetzt, das Kristallwasser anlagert. Dadurch wird in der Regel auch die Filtrierbarkeit des ausgefallenen Salzes verbessert. Bei Verwendung von Natriumnitrit und Essigsäure oder Schwefelsäure zur Freisetzung von salpetriger Säure bieten sich Natriumacetat oder Natriumsulfat aus einem vorhergehenden Ansatz an, die zuvor durch Erhitzen auf 100 bis 200 °C entwässert wurden.

Man kann natürlich auch Wasser aus dem Reaktionsgemisch entfernen, indem man es ihm nach Beendigung der Reaktion durch Destillieren oder Abstrippen entzieht.

Es hat sich in vielen Fällen als vorteilhaft erwiesen, das Reaktionsgemisch vor der Abtrennung des entstandenen Salzes mit einem möglichst unpolaren, inerten Lösungsmittel, wie Methyl-tert.-butylether, Cyclooctan oder Isooctan, zu verdünnen, Das Reaktionsgemisch läßt sich dann besser handhaben und das Salz besser abfiltrieren. Weiterhin hat sich gezeigt, daß dadurch das Nitrosierungsprodukt in reinerer Form erhalten wird. Dadurch wird der Katalysatorbedarf bei der häufig anschließenden katalytischen Hydrierung herabgesetzt.

Das entstandene Salz fällt auch ohne die Verdünnung des Reaktionsgemisches mit bemerkenswerter Reinheit an und kann nach Waschen mit einem geeigneten Lösungsmittel, wie Methyl-tert.-butylether, Cyclohexan und Ethylacetat, und nach Trocknen in anderen Verfahren eingesetzt werden. Schwierig zu entsorgende Salzlösungen fallen bei dem Verfahren nach der Erfindung allenfalls in untergeordneten Mengen an.

Die folgenden Beispiele sollen das Verfahren nach der Erfindung weiter erläutern, nicht aber seinen Anwendungsbereich limitieren.

### Beispiel 1

Zu einer auf 35 bis 40°C gehaltenen gerührten Mischung aus 132,0 g (1,0 Mol) Malonsäuredimethylester, 250,0 g 1,4-Dioxan, 35,6 g Wasser und 80,0 g (1,15 Mol) Natriumnitrit (technisch) wurden innerhalb von 2 Stunden 112,2 g (Essigsäure (96%; 1,8 Mol) getropft. Man ließ 2 Stunden bei 50°C nachreagieren, dann wurden 35 g wasserfreies Natriumacetat sowie 400 g Methyl-tert.-butylether zugegeben. Nach dem Abkühlen auf Raumtemperatur wurde der ausgefallene grobkristalline Feststoff abgesaugt und zweimal mit jeweils 150 ml Methyl-tert.-butylether gewaschen. Filtrat und Waschflüssigkeit wurden bei 60°C im Wasserstrahlvakuum von Leichtsiedern befreit. Es verblieben 186,4 g eines festen Rückstands vom Schmelzbereich 47 bis 56°C. Es handelte sich nach gaschromatischer/massenspektrographischer (GC/MS) Analyse um ein Gemisch aus Hydroxyiminomalonsäuredimethylester und Acetoxyiminomalonsäuredimethylester. Das Produkt enthielt nach GC-Analyse <0,5 FID-Flächenprozent nicht umgesetzten Malonsäuredimethylester.

### Beispiel 2

Zu einer auf 40°C gehaltenen Mischung aus 320,4 g (2,0 Mol) Malonsäurediethylester, 500 g 1,4-Dioxan, 71,2 g entsalztem Wasser und 160 g (2,3 Mol) Natriumnitrit (technisch) wurden innerhalb von 2 Stunden 224,3 g (3,6 Mol) Essigsäure getropft. Man ließ das Reaktionsgemisch 1 Stunde bei 50°C nachreagieren, auf Raumtemperatur abkühlen und impfte die homogene Mischung mit einer Spatelspitze Natriumacetat-trihydrat an, worauf das während der Reaktion gebildete Natriumacetat mit 0 bis 3 Molekülen Kristallwasser grobkristallin ausfiel. Das Gemisch wurde filtriert, der Filterkuchen mit 1,4-Dioxan gewaschen, und die Leichtsieder wurden aus dem Filtrat und der Waschflüssigkeit abdestilliert. Es verblieben 423,0 g eines klaren, hellgelben Öls, das <0,6 FID-Flächenprozent nicht umgesetzten Malonsäurediethylester enthielt. Nach katalytischer Hydrierung und Umkristallisieren des Rohprodukts wurde Acetamidomalonsäurediethylester in einer Ausbeute von 85 %d.Th., bezogen auf eingesetzten Malonsäurediethylester, mit einer Reinheit von >99,8 FID-Flächenprozent erhalten.

### Beispiel 3

Es wurde wie in Beispiel 2 verfahren, jedoch wurden nach Beendigung der Reaktion 70 g wasserfreies Natriumacetat (aus einem vorhergehenden Ansatz durch Filtration und Trocknen im Wasserstrahlpumpenvakuum bei 130°C erhalten) zugesetzt. Dadurch ließ sich das entstandene Natriumacetat leichter als im Beispiel 2 abfiltrieren. Die Ausbeute betrug 417,2 g hellgelbes Öl mit <0,6 FID-Flächenprozent nicht umgesetztem Malonsäurediethylester. Durch katalytische Hydrierung und Umkristallisieren des Rohprodukts wurde Acetamidomalonsäurediethylester mit einer Ausbeute von 85%d.Th., bezogen auf eingesetzten Malonsäurediethylester, und einer Reinheit von >99,8 FID-Flächenprozent erhalten.

### Beispiel 4

Es wurde wie im Beispiel 3 verfahren, doch wurden nach der Zugabe des wasserfreien Natriumacetats zusätzlich 400 g Methyl-tert.-butylether zugefügt. Es blieben 426 g hellgelbes Öl zurück, aus dem durch katalytische Hydrierung und Umkristallisieren des Rohprodukts Acetamidomalonsäurediethylester mit einer Ausbeute von 88%d.Th., bezogen auf eingesetzten Malonsäurediethylester, und einer Reinheit von >99,8 FID-Flächenprozent gewonnen wurde.

### Beispiel 5

Zu einer auf 35°C gehaltenen gerührten Mischung aus 320,4 g (2,0 Mol) Malonsäurediethylester und 160 g (2,3 Mol) Natriumnitrit (technisch) wurde ein Gemisch aus 1,4-Dioxan. Essigsäure und Wasser dosiert, das aus einem vorhergehenden Ansatz durch Abtoppen der Leichtsieder erhalten worden war. Danach wurden 12,0 g Wasser zudosiert, und innerhalb von 2 Stunden wurden 166 g (2,7 Mol) Essigsäure (96%) zugetropft. Man ließ das Gemisch 2 Stunden bei 40°C nachreagieren und arbeitete es auf, wie in Beispiel 4 beschrieben. Es blieben 425 g hellgelbes Öl zurück, aus dem durch katalytische Hydrierung und Umkristallisieren des Rohprodukts Acetamidomalonsäurediethylester mit einer Ausbeute von 86%d.Th., bezogen auf eingesetzten Malonsäurediethylester, und einer Reinheit von >99,8 FID-Flächenprozent gewonnen wurde.

### Beispiel 6

Es wurde wie im Beispiel 2 verfahren, jedoch wurde das 1,4-Dioxan durch Tetrahydrofuran ersetzt. Es blieben 427 g hellgelbes Öl zurück, das <1 FID-Flächenprozent nicht umgesetzten Malonsäurediethylester enthielt.

### Beispiel 7

Es wurde wie im Beispiel 3 verfahren, jedoch wurde das 1,4-Dioxan durch 600 g Ethylacetat ersetzt, die zugesetzte Wassermenge auf 20 g reduziert und die Essigsäure durch 166,6 g Acetanhydrid ersetzt. Es blieben 425 g hellgelbes Öl zurück, aus dem durch katalytische Hydrierung und Umkristallisieren des Rohprodukts Acetamidomalonsäurediethylester mit einer Ausbeute von 85%d.Th., bezogen auf eingesetzten Malonsäurediethylester, und einer Reinheit von >99 FID-Flächenprozent gewonnen wurde.

### Beispiel 8

Es wurde wie im Beispiel 3 verfahren, jedoch wurde das 1,4-Dioxan durch die gleiche Menge Polyethylenglykolether (Molgewicht etwa 500) ersetzt. Man erhielt 921 g einer Lösung, in der die Nitrosierungsprodukte enthalten waren. Sie enthielt <1 FID-Flächenprozent nicht umgesetzten Malonsäurediethylester.

### Beispiel 9

Zu einer auf 35 bis 40°C gehaltenen gerührten Mischung aus 216,0 g (1 Mol) Malonsäurediisobutylester, 250 g 1,4-Dioxan, 35,6 g entsalztem Wasser und 80 g (1,15 Mol) Natriumnitrit (technisch) wurden innerhalb von 2 Stunden 112,2 g Essigsäure (96%) zugetropft. Man ließ das Gemisch 2 Stunden bei 50°C nachreagieren und setzte dann 35,0 g wasserfreies Natriumacetat sowie 200 g Methyl-tert.-butylether zu. Nach dem Abkühlen des Gemisches wurde das ausgefallene grobkristalline Natriumacetat-trihydrat abfiltriert und mit Methyl-tert.-butylether gewaschen. Nach Abdestillieren der Leichtsieder bei 60°C im Wasserstrahlvakuum wurden 258,7 g (88,6%d.Th.) Rückstand erhalten, der überwiegend aus Hydroxyiminomalonsäurediisobutylester bestand. Der Gehalt an nicht umgesetztem Malonsäurediisobutylester betrug 0,4 FID-Flächenprozent.

### Beispiel 10

Zu einer auf 25°C gehaltenen gerührten Mischung aus 113,1 g (1,0 Mol) Cyanessigsäureethylester, 250,0 g 1,4-Dioxan, 35,6 d entsalztem Wasser und 80,0 g (1,15 Mol) Natriumnitrit (technisch) wurden innerhalb von 2 Stunden 57,0 g konzentrierte Schwefelsäure getropft. Man ließ das Gemisch 2 Stunden bei 25°C nachreagieren, fügte dann 400 g Methyltert.butylether zu, kühlte das Gemisch auf Raumtemperatur ab und filtrierte die erhaltene Suspension. Der Filterkuchen wurde zweimal mit jeweils 100 ml Methyl-tert.-butylether gewaschen. Aus dem Filtrat und der Waschflüssigkeit wurden bei 60°C/16mmHg Leichtsieder entfernt. Es blieben 136,7 g ölig-kristalliner Rückstand, der <0,4 FID-Flächenprozent nicht umgesetzten Cyanessigsäureethylester enthielt.

### Beispiel 11

Zu einer auf 35°C gehaltenen gerührten Mischung aus 130,2 g Acetessigsäureethylester, 250 g 1,4-Dioxan, 35,6 g entsalztem Wasser und 80 g Natriumnitrit wurden innerhalb von 2 Stunden 112,2 g Essigsäure (96%; 1,8 Mol) getropft. Man ließ das Gemisch 2 Stunden bei 50°C nachreagieren, setzte der erhaltenen klaren Lösung 200 g Methyl-tert.-butylether zu und ließ das Gemisch auf Raumtemperatur abkühlen. Nach dem Abfiltrieren und Waschen des ausgefallenen Feststoffs wurden das Filtrat und die Waschflüssigkeit bei 60°C im Wasserstrahlvakuum eingeengt. Es blieben 170,4 g (99%d.Th.) Acetoxyiminoacetessigsäureethylester zurück, dessen Gehalt an nicht umgesetztem Acetessigsäureethylester 0,5 FID-Flächenprozent betrug.

### Beispiel 12

Zu einer auf 35°C gehaltenen gerührten Mischung aus 224,0 g Dibenzoylmethan, 250 g 1,4-Dioxan, 35,6 g entsalztem Wasser und 80,0 g Natriumnitrit (technisch) wurden innerhalb von 2 Stunden 112,2 g Essigsäure (96%; 1,8 Mol) dosiert. Man ließ das Gemisch 2 Stunden bei maximal 40°C nachreagieren, fügte 600 g Methyl-tert.-butylether zu und ließ das Gemisch auf Raumtemperatur abkühlen. Nach Abfiltrieren und Waschen des ausgefallenen Natriumacetats wurden das Filtrat und die Waschflüssigkeit bei 60°C im Wasserstrahlvakuum von Leichtsiedern befreit. Es verblieben 216,6 g kristalliner Rückstand mit einem Gehalt an nicht umgesetztem Dibenzoylmethan von <4 FID-Flächenprozent.

### Beispiel 13

Zu einer auf 20°C gehaltenen gerührten Mischung aus 51,0 g Malonsäurediamid, 41,8 g entsalztem Wasser, 250 g 1,4-Dioxan und 19,9 g Natriumnitrit (technisch) wurden innerhalb von 3 Stunden 28,2 g Schwefelsäure getropft. Man ließ 2 Stunden bei 30°C nachreagieren, setzte dem Gemisch 300 ml Ethylacetat und danach 20,0 g wasserfreies Natriumsulfat zu und ließ es auf Raumtemperatur abkühlen. Der ausgefallene Feststoff wurde abfiltriert und mehrmals mit Ethylacetat gewaschen. Aus dem Filtrat und der Waschflüssigkeit wurden unter vermindertem Druck die Leichtsieder entfernt. Als Rückstand verblieb ein Nitrosierungsprodukt mit einem Schmelzbereich von 158°C bis 167°C und einem Gehalt an nicht umgesetztem Malonsäurediamid von <2 FID-Flächenprozent.

### Beispiel 14

Zu einer auf 35°C gehaltenen gerührten Mischung aus 320 g Malonsäurediethylester (2 Mol), 15 g entsalztem Wasser und 160 g Natriumnitrit (2,3 Mol) wurden innerhalb von 2 Stunden 225,0 g Eisessig getropft. Man ließ 2 Stunden bei 50°C nachreagieren, dann wurde das Gemisch filtriert und der Filterkuchen mit etwas Eisessig gewaschen. Das Filtrat und die Waschflüssigkeit wurden bei 60°C im Wasserstrahlvakuum von Leichtsiedern befreit. Als Rückstand verblieben 410,1 g eines klaren, hellgelben Öls, welches nach gaschromatographischer Analyse neben Hydroxyiminomalonsäurediethylester und O-Acetoxyiminomalonsäurediethylester 5 FID-Flächenprozent nicht umgesetzten Malonsäurediethylester enthielt. Das abgetrennte Natriumacetat wurde im Wasserstrahlvakuum bei 60°C getrocknet (Auswaage 160,0g). Es war in entsalztem Wasser vollständig löslich.

### Beispiel 15

Es wurde wie in Beispiel 14 verfahren, jedoch wurden nach Beendigung der Reaktion 800 g Methyl-tert.-butylether zugesetzt, und das Gemisch wurde bei Raumtemperatur filtriert. Es verblieben 420,2 g eines klaren, hellgelben Öls, welches nach gaschromatographischer Analyse neben Hydroxyiminomalonsäurediethylester und O-Acetoxyiminomalonsäurediethylester 5 FID-Flächenprozent nicht umgesetzten Malonsäurediethylester enthielt.

## Patentansprüche

1. Verfahren zur Nitrosierung von C-H-aciden Verbindungen der allgemeinen Formel
X¹-CHR-X², (I)
in der R Wasserstoff oder einen organischen Rest bedeutet und X¹ sowie X² für gleiche oder verschiedene elektronenanziehende Gruppen stehen, durch Umsetzung mit salpetriger Säure, die aus einem Nitrit durch eine andere Säure freigesetzt wird, in Gegenwart von Wasser und eines inerten organischen Lösungsmittels, **dadurch gekennzeichnet**, daß man als inertes organisches Lösungsmittel ein mit Wasser zumindest teilweise mischbares inertes organisches Lösungsmittel oder Lösungsmittelgemisch verwendet und die Mengen dieses Lösungsmittels oder Lösungsmittelgemisches und des Wassers so aufeinander abstimmt, daß der Ausgangsstoff (I), dessen Nitrosierungsprodukt, das Wasser sowie das inerte organische Lösungsmittel oder Lösungsmittelgemisch und zumindest ein Teil des Nitrits und der anderen Säure, die die salpetrige Säure freisetzt, eine homogene flüssige Phase bilden, in der die Nitrosierungsreaktion abläuft und aus der zumindest der größte Teil des bei der Freisetzung der salpetrigen Säure entstehenden Salzes ausfällt.

2. Verfahren nach Anspruch 1, **dadurch gekennzeichnet**, daß in der Formel I X¹ und X² für -COOR, -C(NR)OR, -CONR₂, -CO-R, -CN, -NO₂ oder einen aromatischen Rest stehen, wobei R die für die Formel I angegebene Bedeutung hat.

3. Verfahren nach Anspruch 1 oder 2, **dadurch gekennzeichnet**, daß R für Wasserstoff, einen Alkylrest mit 1 bis 4 Kohlenstoffatomen oder einen Arylrest mit 6 bis 10 Kohlenstoffatomen steht.

4. Verfahren nach einem der Ansprüche 1 bis 3, **dadurch gekennzeichnet**, daß das Nitrit ein Alkalinitrit ist und in einer Menge von 1 bis 1,5 Mol je Mol Verbindung der Formel (I) angewandt wird.

5. Verfahren nach einem der Ansprüche 1 bis 4, **dadurch gekennzeichnet**, daß als Säure, die die salpetrige Säure freisetzt, eine Säure, gegebenenfalls ganz oder zum Teil in Form ihres Anhydrids, verwendet wird, die das Alkalinitrit zumindest teilweise zu protonisieren vermag, und zwar in einer Menge, die mindestens der Menge des eingesetzten Nitrits äquivalent ist.

6. Verfahren nach Anspruch 5, **dadurch gekennzeichnet**, daß die Säure Essigsäure oder Schwefelsäure ist.

7. Verfahren nach einem der Ansprüche 1 bis 6, **dadurch gekennzeichnet**, daß man als mit Wasser zumindest teilweise mischbares inertes organisches Lösungsmittel einen aliphatischen oder cyclischen Ether, einen Carbonsäureester, ein Carbonsäureamid oder die Carbonsäure verwendet, durch die die salpetrige Säure aus einem Nitrit freigesetzt wird.

8. Verfahren nach einem der Ansprüche 1 bis 7, **dadurch gekennzeichnet**, daß man dem Reaktionsgemisch nach Beendigung der Nitrosierungsreaktion einen Stoff zusetzt, der Wasser bindet.

9. Verfahren nach einem der Ansprüche 1 bis 8, **dadurch gekennzeichnet**, daß man dem Reaktionsgemisch nach Beendigung der Nitrosierungsreaktion durch Destillieren oder Abstrippen Wasser entzieht.

## Claims

1. A process for nitrosation of C-H-acidic compounds of the general formula
X¹-CHR-X² (I)
in which R is hydrogen or an organic radical and X¹ and X² are identical or different electron-withdrawing groups, by reaction with nitrous acid, liberated from a nitrite by means of another acid, in the presence of water and of an inert organic solvent, characterized in that the inert organic solvent used is an inert organic solvent or solvent mixture which is at least partially miscible with water and the amounts of this solvent or solvent mixture and of the water are adjusted in relation to one another so that the starting material (I), its nitrosation product, the water and the inert organic solvent or solvent mixture and at least a part of the nitrite and of the other acid which liberates the nitrous acid form a homogeneous liquid phase in which the nitrosation reaction takes place and out of which at least the largest part of the salt which is formed during the liberation of the nitrous acid is precipitated.

2. A process according to claim 1, characterized in that in the formula I X¹ and X² are -COOR, -C(NR)OR, -CONR₂, -CO-R, -CN, -NO₂ or an aromatic radical, where R has the meaning stated for the formula I.

3. A process according to claim 1 or 2, characterized in that R is hydrogen, an alkyl radical having from 1 to 4 carbon atoms or an aryl radical having from 6 to 10 carbon atoms.

4. A process according to any one of claims 1 to 3, characterized in that the nitrite is an alkali metal nitrite and is used in an amount of from 1 to 1.5 mol per mole of compound of the formula (I).

5. A process according to any one of claims 1 to 4, characterized in that the acid used which liberates the nitrous acid is an acid, if desired entirely or partly in the form of its anhydride, which is able at least partially to protonate the alkali metal nitrite, and is used in an amount which is equivalent at least to the amount of nitrite employed.

6. A process according to claim 5, characterized in that the acid is acetic acid or sulfuric acid.

7. A process according to any one of claims 1 to 6, characterized in that the inert organic solvent used which is at least partially miscible with water is an aliphatic or cyclic ether, a carboxylic acid ester, a carboxylic acid amide or the carboxylic acid which liberates the nitrous acid from a nitrite.

8. A process according to any one of claims 1 to 7, characterized in that, after the nitrosation reaction has ended, a material which absorbs water is added to the reaction mixture.

9. A process according to any one of claims 1 to 8, characterized in that, after the nitrosation reaction has ended, water is removed from the reaction mixture by distillation or stripping.

## Revendications

1. Procédé de nitrosation de composés à C-H acides de formule générale :
X¹-CHR.-X² (I)
dans laquelle,
R signifie de l'hydrogène ou un radical organique, et X¹ ainsi que X² représentent des groupes attracteurs d'électrons identiques ou différents,
par réaction avec l'acide nitreux qui est mis en liberté à partir d'un nitzite au moyen d'un autre acide, en présence d'eau et d'un solvant organique inerte,
caractérisé en ce qu'
on utilise comme solvant organique inerte, un solvant organique inerte au moins partiellement miscible a l'eau ou un mélange de solvants et les quantités de ce solvant ou de ce mélange de solvants et d'eau sont mises en accord, les unes avec les autres, de sorte que le produit de départ (I), son produit de nitrosation, l'eau ainsi que le solvant inerte ou le mélange de solvants et au moins une partie du nitrite et de l'autre acide qui met l'acide nitreux en liberté, forment une phase liquide homogène, dans laquelle la réaction de nitrosation se déroule et à partie de laquelle au moins la plus grande partie du sel qui se forme au cours de la libération de l'acide nitreux, précipite.

2. Procédé selon la revendication 1,
caractérisé en ce que
dans la formule (I) X¹ et X² representent -COOR, C(NR)OR, CONR₂, CO-R, CN, NO₂ ou un reste aromatique, dans lequel R a la signification indiquée pour la formule (I).

3. Procédé selon les revendications 1 ou 2,
caractérisé en ce que
R représente de l'hydrogène, un radical alkyle, ayant de 1 à 4 atomes de carbone, ou un reste aryle ayant de 6 à 10 atomes de carbone.

4. Procédé selon l'une des revendications 1 à 3,
caractérisé en ce que
le nitrite est un nitrite de métal alcalin et qu'il est utilisé dans une quantité allant de 1 à 1,5 mol par mol de composé de formule (I).

5. Procédé selon l'une des revendications 1 à 4,
caractérisé en ce que
comme acide qui libère l'acide nitreux, on utilise un acide éventuellement en totalité ou en partie sous forme de son anthydride, qui est capable de protoner le nitrite de métal alcalin au moins en partie et assurément en une quantité qui est équivalente à la-quantité du nitrite mis en oeuvre.

6. Procédé selon la revendication 5,
caractérisé en ce que
l'acide est de l'acide acétique ou de l'acide sulfurique.

7. Procédé selon l'une des revendications 1 à 6,
caractérisé en ce qu'
on utilise comme solvant organique inerte au moins partiellement miscible à l'eau, un éther aliphatique ou cyclique, un ester d'acide carboxylique, un amide d'acide carboxylique, ou l'acide carboxylique à l'aide duquel l'acide nitreux est libéré à partir d'un nitrite.

8. Procédé selon l'une des revendications 1 à 7,
caractérisé en ce qu'
on ajoute au mélange réactionnel après achèvement de la réaction de nitrosation, une substance qui se lie à l'eau.

9. Procédé selon l'une des revendications 1 à 8, caractérisé en ce qu'
on élimine l'eau du mélange réactionnel après achèvement de la réaction de nitrosation par distillation ou par extraction.
